(19) Europäisches Patentamt
European Patent Office
Office européen des brevets

(11) Publication number: **0 076 834**
**B1**

(12) **EUROPEAN PATENT SPECIFICATION**

(45) Date of publication of patent specification: **28.10.87**

(51) Int. Cl.$^4$: **G 01 N 27/16**

(21) Application number: **82901298.8**

(22) Date of filing: **15.04.82**

(86) International application number:
**PCT/DK82/00034**

(87) International publication number:
**WO 82/03689 28.10.82 Gazette 82/26**

# (54) AN APPARATUS FOR ANALYSING COMBUSTION GAS.

(30) Priority: **15.04.81 DK 1722/81**

(43) Date of publication of application:
**20.04.83 Bulletin 83/16**

(45) Publication of the grant of the patent:
**28.10.87 Bulletin 87/44**

(84) Designated Contracting States:
**AT DE FR GB NL**

(56) References cited:
**DE-A-2 850 466**
**DE-B-2 655 954**
**FR-A-2 071 504**
**GB-A-2 005 025**
**SE-A-77 119 873**
**SE-B- 302 215**
**US-A-4 135 381**
**US-A-4 147 513**

(73) Proprietor: **JOHANNSEN, Olaf**
**Sundgade 22**
**DK-6400 Sonderborg (DK)**

(72) Inventor: **JOHANNSEN, Olaf**
**Sundgade 22**
**DK-6400 Sonderborg (DK)**

(74) Representative: **Descourtieux, Philippe et al**
**CABINET BEAU de LOMENIE 55 rue d'Amsterdam**
**F-75008 Paris (FR)**

**EP 0 076 834 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to an apparatus of the type defined in the introductory portion of Claim 1.

Most of the combustibles in combustion gases, which may be exhaust gases from internal combustion engines or flue gases from heating plants, are comprised of carbon monoxide CO and hydrogen $H_2$ which are present in virtually constant quantitative proportions. The same applies to unburned hydrocarbons (CH) with an accuracy acceptable in practice. The rest of the gas, apart from oxygen $O_2$, is either inactive as is the case with nitrogen $N_2$, carbon dioxide $CO_2$ and water vapour $H_2O$, or is not present in any great amounts.

The measurement result produced by the known apparatus of the present type can thus be taken as an expression of the CO content in the combustion gas, and as a matter of fact the apparatus is used in practice for measuring the CO content, e.g. in case of carburetter adjustment. In the combustion the following processes take place:

$$2CO+O_2\rightarrow 2CO_2+68\ \text{kcal.}$$
$$2H_2+O_2\rightarrow 2H_2O+58\ \text{kcal.}$$
$$(CH)_n+mO_2\rightarrow pCO_2+q\ H_2O+x\ \text{kcal.}$$

The amount of heat generated per unit of time determines the resistance of the catalyzing platinum wire and is taken as an expression of the reaction rate of the $CO/O_2$ mixture which at a fixed temperature and pressure and steady state gas mixture is

$$V=k_1\cdot(CO)^2\cdot(O_2),$$

where the concentrations of the reacting gases are stated by brackets, and $k_1$ is a catalyst dependent constant.

If $(O_2)$ is kept constant by accurate dosage of the air volume flow supplied, the reaction rate and thus the reaction heat produced by the catalytic combustion will depend on (CO) alone, i.e. the reaction heat is a measure of (CO). This is obtained by suitable selection of the flow rate of the gas mixture and of the other dependent variables in the combustion system.

Nozzles through which the gases are drawn by a pump can be used for the dosage of the volume flows. An air inlet nozzle of the same size as the gas inlet nozzle gives the $O_2$ amount $\frac{1}{2}\cdot 21\%=10.5\%$ so that an $O_2$ content in the combustion gas of not more than about 2% cannot cause an error exceeding

$$\frac{2\cdot 100}{21}\%=9.5\%$$

at very small CO concentrations of about 0.5%.

The increasing fuel prices have resulted in an ever greater desire for the best possible utilization of the fuel and thus the greatest possible reduction of the content of combustibles in the combustion gas. When a carburetter is adjusted to a lean mixture, i.e. to a petrol/air weight ratio below 1:13, e.g. 1:15, the consumption of petrol falls, while the tractive force will decrease significantly only when the mixture is made even leaner. The leaner the mixture supplied to the engine, the lower the CO content in the exhaust gas; but the oxygen content in the gas will simultaneously increase because of the increasing excess of air which is supplied to the engine. A leaner mixture also causes a greater generation of heat in the engine, and when the oxygen content in the exhaust gas exceeds 3 to 5% by volume, there is a great risk of engine breakdown due to burning of the valves.

Thus, in connection with adjustment of carburetters there is a great need for a measuring facility of not only the content of combustibles, in particular CO, in the exhaust gas, but also of the $O_2$ content to prevent this from getting so great that the exhaust valves of the engine will be endangered.

It is disclosed in DE—A—2 655 954 an apparatus for analysing combustion gases containing combustible components of an essentially known composition, said apparatus comprising a residual combustion gas meter having means to supply a predetermined volume flow of a mixture of combustion gas and air in a specific ratio to a combustion chamber, which contains a combustion catalyzing element, preferably a platinum wire, to generate catalytic combustion of a part of the mixture, and having a measuring bridge to measure the electrical resistance of the combustion catalyzing element as an expression of the content of said combustible components of the combustion gases and said apparatus further including an $O_2$ meter.

Accordingly, an on-off arrangement is disclosed which serves to make the measurement of the combustible content ineffective when the oxygen content is insufficient to sustain combustion. This arrangement provides the measurement of the $O_2$ content for this purpose by electrochemical reaction means.

There is no mutual coupling or transfer of correcting signals from one measuring circuit to the other.

US—A—4 147 513 discloses generally means for measuring the $O_2$ content in an exhaust gas by means

of a resistance type oxygen sensor comprising a combustion chamber and a catalyzing element. The measurement result is used to control the supply of fuel or air to minimize the amount of noxious compounds in exhaust gases. The obtained resulting electrical signal of the resistance type sensor represents only the $O_2$ content by exposing a second resistor which depends only on the temperature. This brings the drawback of having the resistance of the sensor depending not only on the content of $O_2$ but also on the temperature.

There is no combination in the prior art of the electrical signals measuring the CO content and the $O_2$ content in the exhaust gases.

The object of the invention is to provide an apparatus of the present type, within reasonable economic limits, which is capable of measuring not only the content of combustibles but also of the $O_2$ content in the exhaust gas in order to prevent it—as set out above—from getting so high that the exhaust valves of the engine would be endangered.

This object is achieved with an apparatus of the type stated at the outset while discussing the prior art, as further defined in the characterizing portion of Claim 1.

It will be seen from the above expression of the heat generation in the combustion chamber that the generation of heat depends upon both the CO concentration and the $O_2$ concentration. The said correcting signal which is supplied to the $O_2$ meter and depends upon the CO content measured by the residual combustion gas meter, eliminates at any rate essentially the effect exerted on the $O_2$ measurement by the variation of the CO content.

As stated above, however, the $O_2$ content in the exhaust gas causes some inaccuracy in the measurement of the CO content. This inaccuracy can be essentially eliminated by the construction of the invention apparatus.

Where a more accurate measurement of the $O_2$ content is desired, regard must also be had to the contributions to the reaction heat which are due to the combustion of hydrogen $H_2$ and hydrocarbons (CH) in the combustion gas. At no load operation, the reaction heat in the $O_2$ measuring chamber is usually composed of the following approximate contributions:

CO: 82—52%
$H_2$: 12—18%
(CH): 6—30%

and at full load operation:

CO: 86—93%
$H_2$: 10—3%
(CH): 6—4%

Claims 2 to 4 define means for providing a correcting signal which depends upon the content of hydrogen and for correcting the $O_2$ measurement and/or the CO measurement on the basis of this signal, and Claims 5 to 7 define means for providing a correcting signal which depends upon the hydrocarbon content and for correcting the $O_2$ measurement and/or the CO masurement and optionally the $H_2$ measurement on the basis of this signal.

The foregoing description is particularly concerned with the use of the apparatus for the control of exhaust gases from internal combustion engines; however, in a modified version the apparatus can also be used for the control of flue gases from heating plants, e.g. oil burners.

The invention will be explained more fully below with reference to the drawing, in which

Figure 1 is a graph which shows an example of the content of CO and $O_2$ in the exhaust gas from an automobile engine as a function of the air/petrol ratio in the fuel mixture supplied,

Figure 2 is a diagram of an embodiment of the apparatus of the invention,

Figure 3 is a diagram of the electric circuitry incorporated in the apparatus,

Figure 4 is a diagram of another, more accurately measuring embodiment of the apparatus of the invention, and

Figure 5 is a diagram of the electric circuitry incorporated in that apparatus.

In the diagrams conduits for the transport of air and gas are shown by double lines, while electric connecting leads are shown by single lines.

In Figure 1 the weight ratio (in per cent) of air to petrol of the fuel mixture is plotted along the abscissa and the content in per cent of carbon monoxide CO and oxygen $O_2$ in the exhaust gas along the ordinate. The curves shown apply to a specific engine and a specific fuel, but are typical. It will be seen that after a reduction of the CO content to about 0.2—0.3% by volume corresponding to a mix proportion of about 14.6—15.0, no considerable additional reduction is obtained by making the mixture leaner, whereas the content of oxygen increases evenly from this mixture range, accompanied by a corresponding increased heat load on the engine, particularly the exhaust valves. Generally, as has been mentioned previously an oxygen content in the exhaust gas above 3 to 5% by vol. should not be tolerated.

The part of the apparatus of Figure 2 which serves to measure the CO content in exhaust gases is generally known and has a pump 10, which draws the exhaust gases and atmospheric air through filters 11

and 12, respectively, and dosing nozzles 13 and 14, respectively, and through a measuring cell 16 in the form of a combustion chamber. A mixing chamber (not shown) may optionally be inserted between that chamber and the nozzles. The mixture of exhaust gases, air and combustion products is discharged to the atmosphere from the pump 10. The measuring cell 16 contains a platinum wire 15 which catalyzes combustion of the CO of the exhaust gases by means of the $O_2$ content of the air. This combustion can be initiated at a relatively low temperature, such as about 400°C, provided by passing an electric current through the platinum wire. The same platinum wire is incorporated in a measuring bridge 17 which applies a signal to a pointer instrument 18, said signal being indicative of the resistance of the wire and thus of its temperature, which in turn is indicative of the generation of heat per unit of time dependent upon the carbon monoxide concentration (CO). In other words, the pointer instrument 18 can be calibrated directly in vol.% CO.

To measure the $O_2$ content, the apparatus additionally comprises a nozzle 19 and a measuring cell 20 through which a dosed volume flow of exhaust gases is drawn by the pump 10 from the filter 11. The measuring cell 20 is of the same type as the measuring cell 16 described in the foregoing, and its platinum wire 21 is incorporated in a measuring bridge 22 which supplies an output signal to a pointer instrument 23.

As will appear from the previously given expression of the reaction rate

$$V=K_1 \cdot (CO)^2 \cdot (O_2),$$

the reaction heat in the measuring cell 20 depends not only upon the $O_2$ content, but also upon the CO content. The circuit shown in Figure 3 serves primarily to eliminate the effect of the (CO) on the output signal from the measuring bridge 22 and, conversely, also to correct the output signal from the measuring bridge 17 for the error caused by the fact that the mixture supplied to the measuring cell 16 not only contains oxygen introduced with the atmosphere through the nozzle 14, but also the oxygen present in the exhaust gases. The circuit comprises two correcting elements or units 24 and 25 and two signal processing units or operational amplifiers 26 and 27. The first correcting element 24 in series with a rectifier 28 is inserted between the CO measuring bridge 17 and its pointer instrument 18. In additiom to being supplied to the instrument 18, the output signal X from the unit 24 is also applied to the input of the unit 26 which provides an output signal $K_1-X^{-\alpha}$ where $K_1$ and $\alpha$ are adjusting factors. This output signal is supplied to an input of the unit 25 which in series with a rectifier 29 is inserted between the output of the $O_2$ measuring bridge 22, from which it receives a signal $Y^1$, and the pointer instrument 23 of the $O_2$ measuring bridge 22. The unit 25 is adapted to multiply its two input signals by each other to provide an output signal $Y=Y^1 \cdot K_1 \cdot X^{-\alpha}$, which is supplied to the pointer instrument 23 and is freed of the part of the signal $Y^1$ which is due to the (CO) in the exhaust gases so that the instrument 23 can be calibrated directly in vol.% $O_2$.

The output signal Y is also supplied to the fourth unit 27, which provides an output signal $-K_2 \cdot Y$ where $K_2$ is an adjusting factor. This output signal is supplied to an input of the first unit 24 adapted to add it to the signal $X^1$ applied to it from the CO measuring bridge 17. The output signal $X=X^1-K_2Y$ thus generated is supplied to the pointer instrument 18, freed of the effect the $O_2$ content in the exhaust gases has on the output signal $X^1$ from the CO measuring bridge 17.

The apparatus shown in Figure 4 contains similar elements 10 to 16 and 19 to 21 as that of Figure 2 and further two measuring cells 30 and 32 with a platinum wire 31 and 33, respectively, and three dosage nozzles 34, 35 and 36, as well as a regulating valve 37.

The nozzle 34 is connected in series with the measuring cells 16 and 30, and this series connection forms one of three branches which are connected in parallel with each other between the nozzles 13 and 14 at one side and the pump 10 at the other. The second branch is formed by a series connection of the measuring cell 32 and the nozzle 35, and the third branch is formed by the regulating valve 37. The nozzle 19, the measuring cell 20, and the nozzle 36 form an independent series connection between the filter 11 and the pump 10.

Circuit-wise the two series-connected measuring cells 16 and 30 are disposed between the nozzle 34 and the pump 10 and therefore operate at a relatively low pressure, which may e.g. be about 0.506 bar (0.5 atm), while the nozzle 35 is disposed between the pump 10 and the measuring cell 32 which thus operates at a relatively high pressure, which may e.g. be about 0.81 bar (0.8 atm). Also the nozzle 36 is disposed between the pump 10 and the measuring cell 20 to which it is connected; the pressure in this measuring cell may e.g. be about 0.709 bar (0.7 atm).

The series connection of the measuring cells 16 and 30 serves to isolate the part of the reaction heat which is due to the hydrogen content in the exhaust gases. It appears from the kinetic theory of matter that the average speed of the molecules is inversely proportional to the square root of the molecular weight. For CO and $H_2$ having the molecular weights of 28 and 2, respectively, we have

$$\frac{\bar{v}_{H_2}}{\bar{v}_{CO}}=\sqrt{\frac{28}{2}}=3.74$$

The thermal conductivity of a gas mixture is due to the fact that energy-rich molecules by their thermal

movements diffuse out between less energy-rich molecules, it being presupposed that the mixing does not simultaneously take place by macroscopic turbulence. From the kinetic theory of matter we have:

$$p \cdot V = \tfrac{1}{3}Nm\bar{v}^2, = \tfrac{1}{3}M\bar{v}^2$$

where

p=pressure
V=volume
N=number of molecules
m=mass of a molecule
$\bar{v}$=average speed of the molecules and
M=molecular weight

The above equation gives

$$\sqrt{\bar{v}^2} = \sqrt{\frac{3pV}{Nm}} = \sqrt{\frac{3RT}{M}}$$

(where R is the gas constant and T is the absolute temperature) i.e. the average speed of the molecules is inversely proportional to the square root of the molecular weight. For CO and $H_2$ having the molecular weights of 28 and 2, respectively, we have:

$$\frac{\bar{v}H_2}{\bar{v}CO} = \sqrt{\frac{28}{.2}} = 3.74.$$

Since according to Graham's law the diffusion rate is proportional to the average speed, $H_2$ will diffuse 3.74 times as far as CO in the same period of time. However, the fast $H_2$ molecules have more collisions than the CO molecules in the same period of time so the random migration of the $H_2$ molecules is therefore actually somewhat shorter than would be expected from the kinetic theory of matter alone.

Similarly, for (CH) we have:

$$\frac{\bar{v}(CH)}{\bar{v}CO} = \sqrt{\frac{28}{36.2}} = 0.86,$$

(CH) being here conceivably composed of:

11.5% $CH_4$
2.1% $C_2H_6$
13.6% $C_2H_4$
56.1% $C_3H_8$
9.9% $C_2H_2$
6.8% $C_3H_6$

which gives an average molecular weight of 36.2.

It is now endeavoured to measure the reaction heat of a catalytic combustion at such a low pressure as will make the dependence of the reaction rate upon the supply of matter emerge. Suppose that a boundary layer is disposed just above the catalyst surface, the molecule movements parallel with the surface will predominantly be produced by the macroscopic translation energy of the ensemble, while the movement in the normal direction of the surface may be expected to be caused mainly by the thermal molecule diffusion. Therefore, in principle it must be possible to measure the diffusion rate of a gas that reacts on the catalyst surface by connecting the two reaction chambers in series and comparing their reaction heat.

For a gas which when burned gives an enthalpy change $\Delta H$ J/mol, the reaction heat will in this case be proportional to $\Delta H/\sqrt{M}$ and also depend upon the temperature of the catalyst, upon the binding energy between gas molecule and catalyst, upon the migration capacity of the gas molecules and upon their ability to stay on the catalyst surface until they have reacted.

It may reasonably be expected that a relatively large part of the fast $H_2$ molecules react in the front reaction chamber as opposed to the more slowly diffusing CO and (CH) molecules. Thus, relatively much $H_2$ is spent in the front chamber, and this is shown in the subsequent chamber by a relative minor reaction heat owing to the great $H_2$ consumption of the front chamber.

As appears from the Tables 1 to 4 below, in which limits for normally occurring concentrations of $H_2$, CO and (CH) are used, it may be expected that the difference between the reaction heat of the two reaction chambers is predominantly to be ascribed to $H_2$ and CO reactions.

TABLE 1

| | M(g) | Enthalpy $\Delta H$ kJ/mol | Reaction heat expressed as: $\frac{\Delta H}{\sqrt{M}}$ |
|---|---|---|---|
| $H_2$ | 2 | 242 | 171 |
| CO | 28 | 283 | 53.5 |
| (CH) | 36.2 | 2043 | 339 |
| NO | 30 | 56 | 10.2 |

TABLE 2
No load operation

| | Conc. c vol.% | $c \cdot \frac{\Delta H}{\sqrt{M}}$ | % rel. share |
|---|---|---|---|
| $H_2$ | 0.5—4 | 85—684 | 33.3—46.0 |
| CO | 3—10 | 160—535 | 62.8—35.9 |
| (CH) | 0.03—0.8 | 10.2—271 | 3.9—18.1 |

TABLE 3
Full load operation, low r.p.m.

| | Conc. c vol.% | $c \cdot \frac{\Delta H}{\sqrt{M}}$ | % rel. share |
|---|---|---|---|
| $H_2$ | 0.2—1 | 34.2—171 | 17.0—27.8 |
| CO | 3—8 | 160—428 | 79.6—69.5 |
| (CH) | 0.002—0.05 | 6.8—17.8 | 3.4—2.8 |

TABLE 4
Full load operation, high r.p.m.

| | Conc. c vol.% | $c \cdot \frac{\Delta H}{\sqrt{M}}$ | % rel. share |
|---|---|---|---|
| $H_2$ | 0.1—0.2 | 17.1—34.2 | 23.1—11.0 |
| CO | 1—5 | 53.5—267 | 72.3—86.0 |
| (CH) | 0.01—0.03 | 3.4—10.2 | 4.6—3.0 |

Other occurring reactions are:

$$2NO + \left\{ \begin{matrix} 2H_2 \\ 2CO \end{matrix} \right\} \rightarrow N_2 \left\{ \begin{matrix} 2H_2O \\ 2CO_2 \end{matrix} \right\}$$

and the secondary reaction

$$2NO + 5H_2 \rightarrow 2NH_3 + 2H_2O$$

which are both catalysed by Pt. Since these reactions only take place at $\lambda<1$ where $\lambda$ is the equivalent air/fuel ratio, they have no practical importance in connection with $O_2$ measurements.

The reaction

$$CO+H_2O \rightleftarrows CO_2+H_2$$

proceeds very slowly, and the reaction

$$CH_x+H_2O \rightarrow (1+\frac{x}{2})H_2+CO_2$$

proceeds only at extremely active Pt.

Thus, an approximate determination of the $CO/H_2$ ratio of the gas may be performed, and as the total reaction heat is known as well, figures for the concentration of CO and $H_2$ can be obtained which, however, are vitiated by errors, caused e.g. by the presence of (CH).

As mentioned in the opening paragraphs, an accurate $O_2$ measurement requires not only knowledge of the CO and $H_2$ concentrations, but also of the (CH) concentration, which should preferably be measured without any phase displacement in terms of time with respect to the other measurements. Also, it would be advantageous to use, as far as possible, the same principle—catalytic combustion—for this measurement as for the others. The next task is then to determine such changes in the state of the gas as will now make the characteristics of (CH) emerge.

The equilibrium constants for the CO and $H_2$ reactions are:

$$K_{CO}=\frac{[CO_2]^2}{[CO]^2\cdot[O_2]} \qquad (\frac{2}{3})$$

$$K_{H_2}=\frac{[H_2O]^2}{[H_2]^2\cdot[O_2]} \qquad (\frac{2}{3})$$

and for the (CH) reactions:

$$K_{CH_4}=\frac{[CO_2]\cdot[H_2O]^2}{[CH_4]\cdot[O_2]^2} \qquad (\frac{3}{3})$$

$$K_{C_2H_6}=\frac{[CO_2]^4\cdot[H_2O]^6}{[C_2H_6]^2\cdot[O_2]^7} \qquad (\frac{10}{9})$$

$$K_{C_2H_4}=\frac{[CO_2]^2\cdot[H_2O]^2}{[C_2H_4]\cdot[O_2]^3} \qquad (\frac{4}{4})$$

$$K_{C_3H_8}=\frac{[CO_2]^3\cdot[H_2O]^4}{[C_3H_8]\cdot[O_2]^5} \qquad (\frac{7}{6})$$

$$K_{C_2H_2}=\frac{[CO_2]^4\cdot[H_2O]^2}{[C_2H_2]^2\cdot[O_2]^5} \qquad (\frac{6}{7})$$

$$K_{C_3H_6}=\frac{[CO_2]^6\cdot[H_2O]^6}{[C_3H_6]^2\cdot[O_2]^9} \qquad (\frac{12}{11})$$

where the exponential sums are stated in the subsequent brackets. It will be seen that in case of equilibrium a sufficient increase in pressure makes the two first-mentioned reactions go to the right while the (CH) reactions mainly go to the left as $C_3H_8$ alone constitutes more than half of the hydrocarbons.

There is no equilibrium in a reaction chamber, but owing to the use of a catalyst and the presence of a high temperature it may be assumed that at least a certain equilibrium is provided directly at the catalyst surface if only the concentrations, i.e. the pressure, are sufficient.

All reactions will produce a greater reaction heat in a chamber with a greater pressure for the mere

reason that now more molecules react, but a greater reaction heat increase is to be expected for CO and $H_2$ than for (CH) because of the concentration dependence of the reaction rates.

When the pressure is doubled, the difference in the reaction heat $q_r$ between a high pressure chamber and a low pressure chamber will be proportional to

$$\Delta H \cdot \left(\frac{q}{p}\right)^2,$$

where q and p are the exponential sum in the counter and denominator, respectively, of the equilibrium constant.

Hence

$$\Delta q_r(CO) \text{ is proportional to } 283 \cdot \left(\frac{3}{2}\right)^2 = 640$$

$$\Delta q_r(H_2) \text{ is proportional to } 242 \cdot \left(\frac{2}{2}\right)^2 = 545$$

$$\Delta q_r(CH) \text{ is proportional to } 2043 \cdot \left(\frac{6}{7}\right)^2 = 1500, \text{ applying to } C_3H_8$$

and

$$\frac{\Delta q_r(CO)}{\Delta q_r(C_3H_8)} = \frac{640}{1500} = 0.43$$

In comparison, measurements performed have given

$$\frac{\Delta q_r(CO)}{\Delta q_r(C_3H_8)} = \frac{1.18}{2.51} = 0.47,$$

which must be said to tally sufficiently well with the theory.

The circuit shown in Figure 5 has four measuring bridges 40—43 to measure the resistances of the platinum wires of the four measuring cells 16, 30, 32 and 20 and to provide signals $X_1$, $X_2$, $X_3$ and $X_4$, respectively, representing these resistances and thus the reaction heat in the measuring cells. The output signal $X_1$ from the bridge 40 is applied to an operational amplifier 44 supplied with another three signals which are indicative of the hydrogen, hydrocarbon and oxygen content, respectively, of the combustion gas. The operational amplifier 44 processes the signals supplied so as to provide an output signal representing the CO content corrected for the three factors mentioned. This output signal is passed through a rectifier 45 to a pointer instrument 46 calibrated in vol.% CO.

The output signal $X_2$ from the bridge 41 is supplied to an operational amplifier 47 which also receives a signal $K_1 \cdot X_1$ from a potentiometer 48 connected to the output of the bridge 40, to generate an output signal $K_1 \cdot X_1 - X_2$, which is supplied to a computing unit 49 together with a version of the signal $X_1$ modified by a circuit 50. The computing unit 49 provides an output signal

$$\frac{K_1 \cdot X_1 - X_2}{X_1}$$

which is supplied to a multiplier 51 that multiplies the signal by $K_2$. The signal

$$\frac{K_1 \cdot X_1 - X_2}{X_1} \cdot K_2$$

thus produced is supplied to an operational amplifier 52 to which are supplied additional signals indicative of the CH, O and CO content, respectively. The operational amplifier 52 is designed to process and combine the signals supplied so as to provide an output signal which represents the hydrogen content and is corrected for errors caused by CH, O and CO.

The signals indicative of the CO and H provided are supplied to their respective potentiometers 53 and 54 to provide signals $K_3(CO)$ and $K_4(H)$, respectively, which are supplied to an operation amplifier 55 that

provides an output signal $[K_3(CO)+K_4(H)]K_5$. This signal together with the output signal $X_3$ from the measuring bridge 42 is applied to an operational amplifier 56, which provides an output signal $X_3-[K_3(CO)+K_4(H)]K_5$ to be supplied to another operation amplifier 57, to which are also supplied signals indicative of the CO, H and O content; the operational amplifier 57 is designed to process and combine the signals supplied so as to provide an output signal which is indicative of the CH content in the combustion gas and is corrected for false contributions.

The output signal $X_4$ from the measuring bridge 43 is supplied to a multiplier 58 which multiplies it by $K_6$, and the signal $K_6 \cdot X_4$ thus produced is applied to a computing unit 59. This unit also receives a signal $(CO)^2+(H)^2+(CH)$ provided by an addition circuit 60 by addition of the three output signals from two computing units 61 and 62 and an operational amplifier 63 to which are supplied the signals CO, H, and CH, respectively. The computing unit 59 provides an output signal

$$\frac{K_6 \cdot X_4}{(CO)^2+(H)^2+(CH)},$$

which represents the oxygen content and is corrected for errors caused by the content of CO, H and CH. This signal is passed through a rectifier 64 to a pointer instrument 65 calibrated in vol.% $O_2$.

The practical details of the apparatus of the invention can be designed in other ways than those shown in the drawing and described in the foregoing. Thus, e.g. the hydrocarbon content might optionally be measured by two series-connected catalyst/combustion chambers analogously with the procedure described for hydrogen.

**Claims**

1. An apparatus for analysing combustion gases containing combustible components of an essentially known composition, said apparatus comprising a residual combustion gas meter having means (13, 14) to supply a predetermined volume flow of a mixture of combustion gas and air in a specific ratio to a first combustion chamber (16), which contains a first combustion catalysing element (15), preferably a platinum wire, to generate catalytic combustion of a part of the mixture, and having a first measuring bridge (17) to measure the electrical resistance of the first combustion catalysing element (15) as an expression of the content of said combustible components of the combustion gases, and said apparatus further including an $O_2$ meter, characterized in that said $O_2$ meter comprises means (19) to supply a predetermined volume flow of the combustion gas to a second combustion chamber (20) including a second combustion catalysing element (21), and comprises a second measuring bridge (22) to measure the resistance of said second combustion catalysing element (21), the apparatus further comprising means (26) to produce a correcting signal dependent upon the output signal of the residual combustion gas meter, means (27) to produce a correcting signal dependent upon the output signal of the $O_2$ meter, a first correcting element (25, 59), which is supplied with the output signal from the second measuring bridge (22) as well as the correcting signal dependent upon the output signal of the residual combustion gas meter and is designed to combine these signals to provide an output signal which depends essentially only upon the $O_2$ content in the gas, and a second correcting element (24, 44) which is supplied with the output signal from the first measuring bridge (17) as well as the correcting signal dependent upon the output signal of the $O_2$ meter and is designed to combine these signals to provide an output signal which depends essentially only upon the content of combustible components in the gas.

2. An apparatus according to Claim 1, characterized by further comprising means (30, 31, 41 and 47—52) to provide a correcting signal depending upon the hydrogen content in the combustion gas, and means (62, 60) to supply said hydrogen dependent signal to the first and/or second correcting element(s) (59 and/or 44).

3. An apparatus according to Claim 2, characterized in that the means to provide the hydrogen dependent signal comprise a third combustion chamber (30) which is connected in series with the first combustion chamber (16) and comprises a third combustion catalysing element (31), means (41) to provide a signal ($X_2$) dependent upon the electrical resistance of said third combustion catalysing element (31), and means (47, 48) to combine said signal with the signal ($X_1$) indicative of the electrical resistance of the first combustion catalysing element (15), to provide a resulting signal indicative of the hydrogen content.

4. An apparatus according to Claim 3, characterized in that the said resulting signal is supplied to a third correcting element (52), which also receives the output signals from the first and second correcting elements (59, 44) to provide a corrected hydrogen dependent correcting signal.

5. An apparatus according to any of the preceding claims, characterized by further comprising means (32, 33, 42 and 53—57) to generate a correcting signal depending upon the hydrocarbon content in the combustion gas, and means (60, 63) to supply said hydrocarbon dependent correcting signal to the first and/or second correcting element(s) (59 and/or 44) and optionally to the third correcting element (52).

6. An apparatus according to Claim 5, characterized in that the means to generate the hydrocarbon dependent signal comprise a fourth combustion chamber (32) equipped with a fourth combustion catalysing element (33), said combustion chamber receiving a predetermined volume flow of an air and

combustion gas mixture and working at a higher pressure than the first measuring chamber (16), means (42) to provide a signal depending upon the resistance of the fourth combustion catalysing element (33), and means (53—56) to combine said signal with the output signals of the second and third correcting elements (44, 52) so as to provide a resulting signal indicative of the hydrocarbon content.

7. An apparatus according to Claim 6, characterized in that the resulting signal indicative of the hydrocarbon content is supplied to a fourth correcting element (57), which also receives the output signals from the first, second and third correcting elements (59, 44, 52) to provide a corrected hydrocarbon dependent signal.

**Patentansprüche**

1. Vorrichtung zur Analyse von Verbrennungsgasen, die verbrennbare Komponenten mit im wesentlicher bekannter Zusammensetzung enthalten, welche Vorrichtung einen Resverbrennungsgasmesser enthält, der Mittel (13, 14) zur Zufuhr eines vorbestimmten Volumenstroms einer Mischung von Verbrennungsgas und Luft in einem spezifischen Verhältnis zu einer ersten Verbrennungskammer (16) aufweist, welche ein erstes verbrennungskatalysierendes Element (15) enthält, vorzugsweise einen Platindraht, um katalytische Verbrennung eines Teiles der Mischung zu bewirken, und eine erste Meßbrücke (17) zum Messen des elektrischen Widerstandes des ersten verbrennungskatalysierenden Elements (15) als Ausdruck des Gehaltes der verbrennbaren Komponenten der Verbrennungsgase hat, welche Vorrichtung weiterhin ein $O_2$-Meßgerät aufweist, dadurch gekennzeichnet, daß dieses $O_2$-Meßgerät Mittel (19) zur Zufuhr eines vorherbestimmten Volumensstromes des Verbrennungsgases zu einer zweiten Verbrennungskammer (20) einschließlich eines zweiten verbrennungskatalysierenden Elements (21) vorsieht, welche eine zweite Meßbrücke (22) zur Messung des Widerstandes dieses zweiten verbrennungskatalysierenden Elementes (21) aufweist, wobei die Vorrichtung weiters Mittel (26) zur Erzeugung eines vom Ausgangssignal des Restverbrennungsgasmeßgeräts abhängigen Korrektursignals aufweist und Mittel (27) zum Erzeugen eines vom Ausgangssignal des $O_2$-Meßgeräts abhängigen Korrektursignals, eine erstes Korrekturelement (25, 59), welches mit dem Ausgangssignal der zweiten Meßbrücke (22) und auch dem Korrektursignal, das vom Ausgangssignal des Restverbrennungsgasmeßgerätes abhängig ist, versorgt wird und zur Kombination dieser beiden Signale bestimmt ist, um eine Ausgangssignal vorzusehen, welches im wesentlichen nur vom $O_2$-Gehalt im Gas abhängt, und eine zweites Korrekturelement (24, 44), welches mit dem Ausgangssignal von der ersten Meßbrücke (17) und dem Korrektursignal, das vom Ausgangssignal des $O_2$-Meßgerätes abhängt, versorgt wird und zur Kombination dieser beiden Signale zu einem Ausgangssignal geschaffen ist, welches im wesentlichen nur vom Gehalt der verbrennbaren Komponenten im Gas abhängig ist.

2. Vorrichtung nach Anspruch 1, dadurch gekennzeichnet, daß sie weiters Mittel (30, 31, 41 und 47—52) zur Vorsehung eines Korrektursignals aufweist, das vom Wasserstoffgehalt im Verbrennungsgas abhängt, sowie Mittel (62, 60), um dieses wasserstoffabhängige Signal zum ersten und/oder zweiten Korrekturelement (59 und/oder 44) zu leiten.

3. Vorrichtung nach Anspruch 2, dadurch gekennzeichnet, daß die Mittel zur Vorsehung des wasserstoffabhängigen Signals eine dritte Verbrennungskammer (30) aufweisen, die in Serie mit der ersten Verbrennungskammer (16) verbunden ist, und ein drittes verbrennungskatalysierendes Element (31) aufweist, sowie Mittel (41) um ein Signal ($X_2$) vorzusehen, welches vom elektrischen Widerstand des dritten verbrennungskatalysierenden Elementes (31) abhängt, sowie Mittel (47, 48), um dieses Signal mit dem Signal ($X_1$) zu kombinieren, des den elektrischen Widerstand des ersten verbrennungskatalysierenden Element (15) anzeigt, um so ein Signal zu erzeugen, das den Wasserstoffgehalt anzeigt.

4. Vorrichtung nach Anspruch 3, dadurch gekennzeichnet, daß das resultierende Signal einem dritten Korrekturelement (52) zugeführt wird, welches auch die Ausgangssignale vom ersten und zweiten Korrekturelement (59, 44) empfängt, um so ein korrigiertes, wasserstoffabhängiges Korrektursignal zu erhalten.

5. Vorrichtung nach einem der vorhergehenden Ansprüche, dadurch gekennzeichnet, daß sie weiters Mittel (32, 33, 42 und 53—57) aufweist zur Erzeugung eines vom Kohlenwasserstoffgehalt im Verbrennungsgas abhängigen Korrektursignals, und Mittel (60, 63), um dieses kohlenwasserstoffabhängige Korrektursignal zum ersten und/oder zweiten Korrekturelement (59 und/oder 44) und gegebenenfalls zum dritten Korrekturelement (52) zu leiten.

6. Vorrichtung nach Anspruch 5, dadurch gekennzeichnet, daß die Mittel zum Erzeugen des kohlenwasserstoffabhängigen Signals eine vierte Verbrennungskammer (32) aufweisen, die mit einem vierten verbrennungskatalysierenden Element (33) ausgerüstet ist und die einen vorbestimmten Volumsstrom einer Luft-Verbrennungsgasmischung empfängt und bei einem höheren Druck als die erste Meßkammer (16) arbeitet, sowie Mittel (42) um eine Signal, das vom Widerstand des vierten die Verbrennung katalysierenden Elements (33) abhängt, zu liefern, und Mittel (53—56), um dieses Signal mit den Ausgangssignalen des zweiten und dritten Korrekturelementes (44, 52) zu kombinieren, um so ein resultierendes Signal vorzusehen, das den Kohlenwasserstoffgehalt anzeigt.

7. Vorrichtung nach Anspruch 6, dadurch gekennzeichnet, daß das resultierende, den Kohlenwasserstoffgehalt anzeigende Signal zu einem vierten Korrekturelement (57) geleitet wird, das auch

die Ausgangssignale vom ersten, zweiten und dritten Korrekturelement (59, 44 und 52) empfängt, um so ein korrigiertes kohlenwasserstoffabhängiges Signal vorzusehen.

**Revendications**

1. Appareil pour analyser des gaz de combustion contenant des composants combustibles d'une composition sensiblement connue, ledit appareil comprenant un dispositif de mesure du gaz de combustion résiduel comportant des moyens (13, 14) pour envoyer un débit en volume prédéterminé d'un mélange de gaz de combustion et d'air dans un rapport spécifique dans une première chambre de combustion (16), qui contient un premier élément de catalyse de combustion (15), de préférence un fil de platine, pour produire la combustion catalytique d'une partie du mélange et comportant un premier pont de mesure (17) pour mesurer la résistance électrique du premier élément de catalyse de combustion (15) comme expression de la teneur en lesdits composants combustibles des gaz de combustion et ledit appareil contenant en outre un dispositif de mesure de $O_2$, caractérisé en ce que ledit dispositif de mesure de $O_2$ comprend des moyens (19) pour envoyer un débit en volume prédéterminé du gaz de combustion dans une seconde chambre de combustion (20) contenant un second élément de catalyse de combustion (21) et comprend un second pont de mesure (22) pour mesurer la résistance dudit second élément de catalyse de combustion (21), l'appareil comprenant en outre des moyens (26) pour produire un signal de correction dépendant du signal de sortie du dispositif de mesure du gaz de combustion résiduel, des moyens (27) pour produire un signal de correction dépendant du signal de sortie du dispositif de mesure de $O_2$, un premier élément de correction (25, 59) qui reçoit le signal de sortie du second pont de mesure (22) ainsi que le signal de correction dépendant du signal de sortie du dispositif de mesure du gaz de combustion résiduel et qui est destiné à combiner ces signaux pour donner un signal de sortie qui ne dépend pratiquement qu de la teneur en $O_2$ dans le gaz et un second élément de correction (24, 44), qui reçoit le signal de sortie du premier pont de mesure (17) ainsi que le signal de correction dépendant du signal de sortie du dispositif de mesure de $O_2$ et qui est destiné à combiner ces signaux pour donner un signal de sortie qui ne dépend pratiquement que de la teneur en composants combustibles dans le gaz.

2. Appareil selon la revendication 1, caractérisé en ce qu'il comprend en outre des moyens (30, 31, 41 et 47—52) pour donner un signal de correction dépendant de la teneur en hydrogène dans le gaz de combustion, et des moyens (62, 60) pour envoyer ledit signal dépendant de l'hydrogène au premier et/ou au second élément(s) de correction (59 et/ou 44).

3. Appareil selon la revendication 2, caractérisé en ce que les moyens pour donner le signal dépendant de l'hydrogène consistent en une troisième chambre de combustion (30) qui est reliée en série avec la première chambre de combustion (16) et comprend un troisième élément de catalyse de combustion (31), des moyens (41) pour donner un signal ($X_2$) dépendant de la résistance électrique dudit troisième élément de catalyse de combustion (31) et des moyens (47, 48) pour combiner ledit signal avec le signal ($X_1$) indiquant la résistance électrique du premier élément de catalyse de combustion (15) pour donner un signal résultant indiquant la teneur en hydrogène.

4. Appareil selon la revendication 3, caractérisé en ce que ledit signal résultant est envoyé à un troisième élément de correction (52), qui reçoit également les signaux de sortie du premier et du second éléments de correction (59, 44) pour donner un signal de correction dépendant de l'hydrogène corrigé.

5. Appareil selon l'une quelconque des revendications précédentes, caractérisé en ce qu'il comprend en outre des moyens (32, 33, 42 et 53—57) pour produire un signal de correction dépendant de la teneur en hydrocarbures dans le gaz de combustion et des moyens (60, 63) pour envoyer ledit signal de correction dépendant des hydrocarbures au premier et/ou au second élément(s) de correction (59 et/ou 44) et facultativement au troisième élément de correction (52).

6. Appareil selon la revendication 5, caractérisé en ce que les moyens pour produire le signal dépendant des hydrocarbures comprennent une quatrième chambre de combustion (32) munie d'un quatrième élément de catalyse de combustion (33), ladite chambre de combustion recevant un débit en volume prédéterminé d'un mélange d'air et de gaz de combustion et fonctionnant sous une pression supérieure à celle de la première chambre de mesure (16), des moyens (42) pour donner un signal dépendant de la résistance du quatrième élément de catalyse de combustion (33) et des moyens (53—56) pour combiner ledit signal avec les signaux de sortie du second et du troisième éléments de correction (44, 52) de manière à fournir un signal résultant indiquant la teneur en hydrocarbures.

7. Appareil selon la revendication 6, caractérisé en ce que le signal résultant indiquant la teneur en hydrocarbures est envoyé au quatrième élément de correction (57) qui reçoit également les signaux de sortie du premier, du second et du troisième élément de correction (59, 44, 52), pour fournir un signal corrigé dépendant des hydrocarbures.

FIG. 1

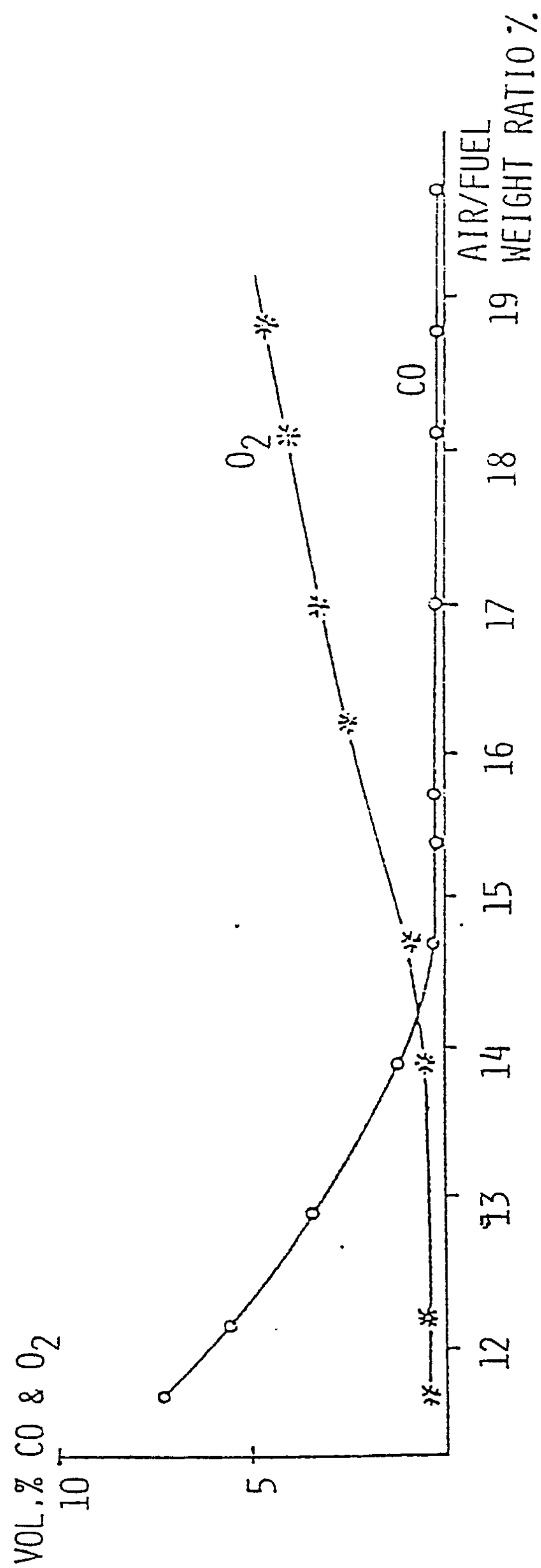

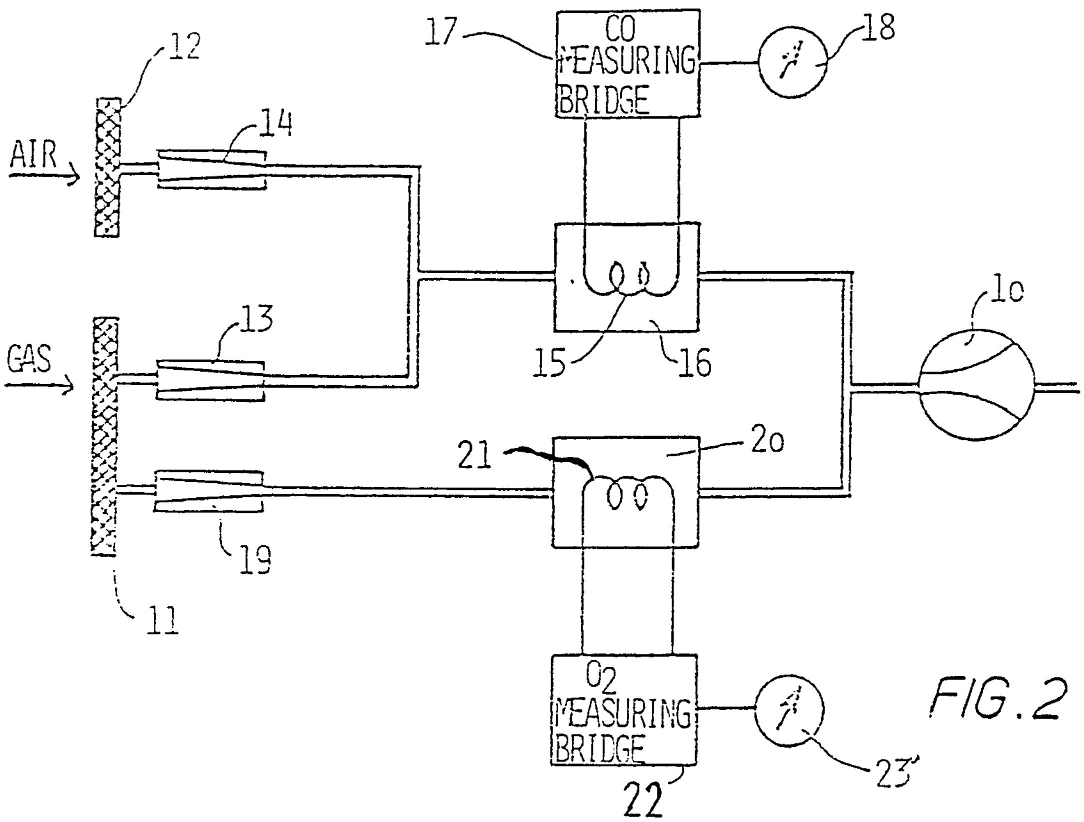

CO MEASURING BRIDGE
17
18
12
14
AIR
GAS
13
15
16
10
21
20
19
11
O2 MEASURING BRIDGE
22
23


FIG. 2

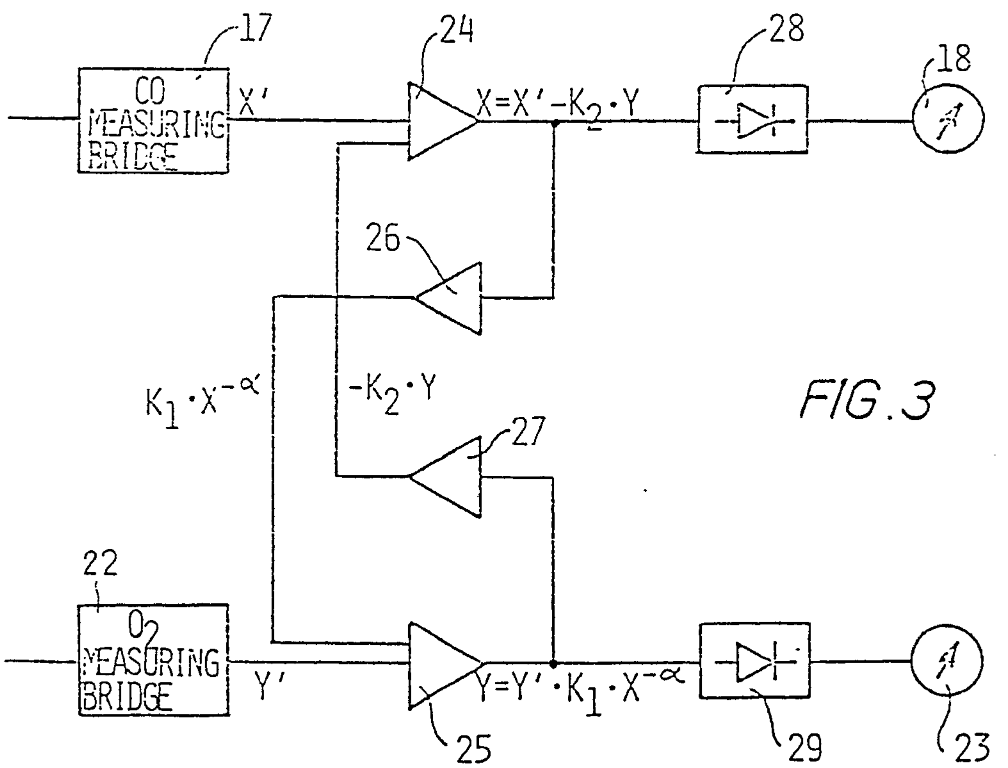

17
CO MEASURING BRIDGE
X'
24
X=X'-K2·Y
28
18
26
K1·X^-α
-K2·Y
27
22
O2 MEASURING BRIDGE
Y'
Y=Y'·K1·X^-α
25
29
23


FIG. 3

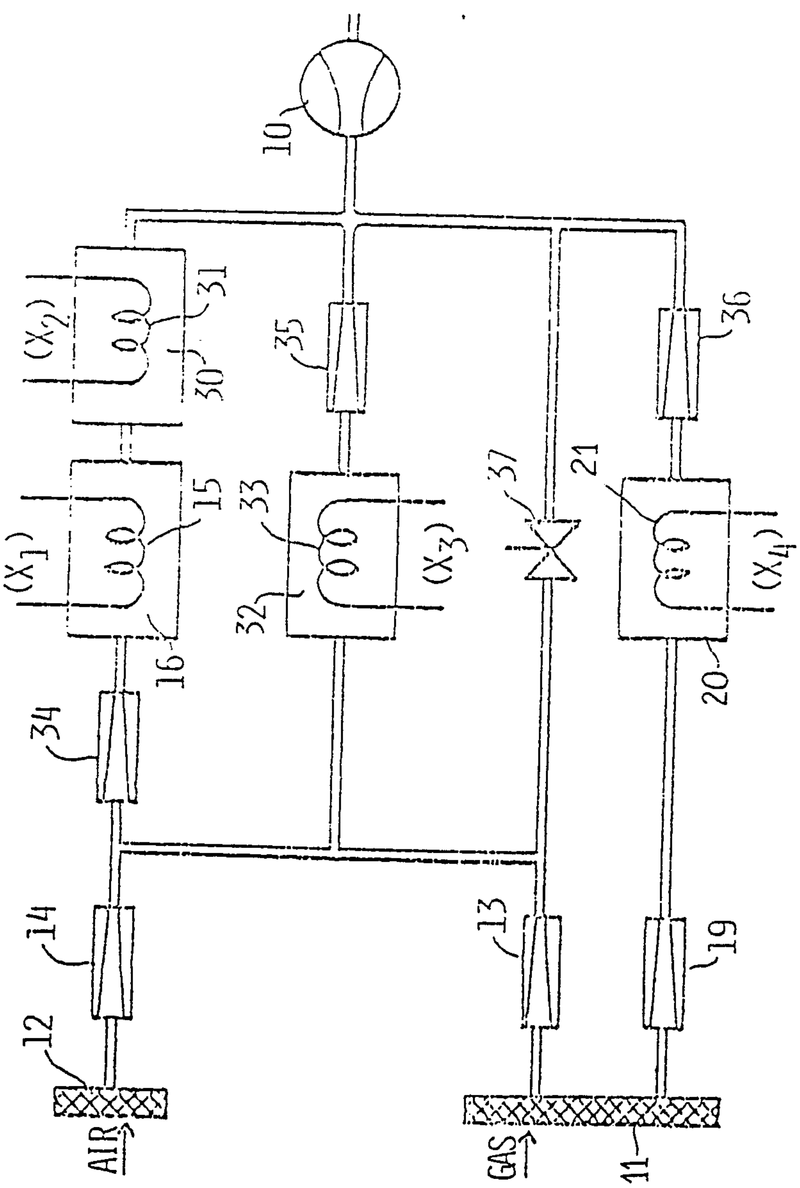
10
31
30
35
36
15
33
37
21
16
32
20
34
14
13
19
12
11
AIR
GAS
($X_1$)
($X_2$)
($X_3$)
($X_4$)

FIG. 4

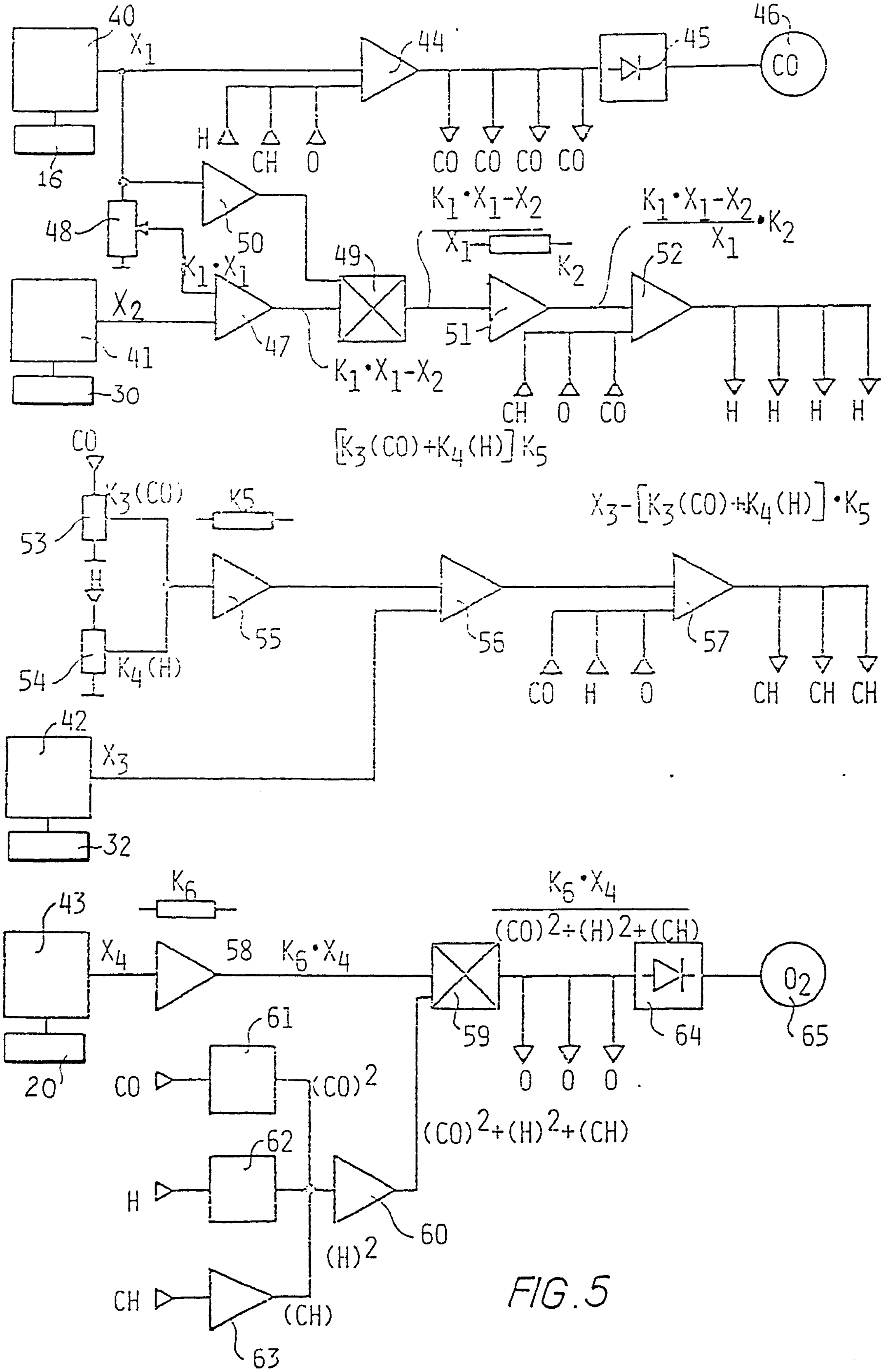
40
X1
44
45
46
CO
H
CH
O
CO CO CO CO
16
48
50
K1•X1-X2
X1
K2
K1•X1-X2 / X1 •K2
52
49
K1•X1
X2
47
51
41
K1•X1-X2
30
CH O CO
H H H H
[K3(CO)+K4(H)]K5
CO
K3(CO)
K5
X3-[K3(CO)+K4(H)]•K5
53
H
55
56
57
54
K4(H)
CO H O
CH CH CH
42
X3
32
K6
K6•X4 / (CO)2+(H)2+(CH)
43
X4
58
K6•X4
O2
59
64
65
20
61
CO
(CO)2
O O O
(CO)2+(H)2+(CH)
62
H
60
(H)2
CH
(CH)
63

FIG. 5